# EUROPEAN PATENT SPECIFICATION

(11) **EP 1 664 005 B1**
(45) Date of publication and mention of the grant of the patent: **09.02.2011**
(21) Application number: 04787171.0
(22) Date of filing: 20.09.2004
(51) Int. Cl.: C07D 277/24, C07D 277/56, C07D 417/12, C07D 417/04, C07D 417/14, A61K 31/426, A61K 31/4439, A61P 25/00

(54) **THIAZOLE DERIVATIVES AS CANNABINOID RECEPTOR MODULATORS**
THIAZOLDERIVATE ALS MODULATOREN DES CANNABINOIDREZEPTORS
UTILISATION DE DERIVES DU THIAZOLE COMME MODULATEURS DES RECEPTEURS CANNABINOIDES

(30) Priority: 19.09.2003 EP 03078309; 22.09.2003 US 504212 P
(43) Date of publication of application: 07.06.2006
(73) Proprietor: Abbott Healthcare Products B.V., 1381 CP Weesp (NL)
(72) Inventor: LANGE, Josephus H.M., NL-1381 CP Weesp (NL); KRUSE, Cornelis G., NL-1381 CP Weesp (NL); VAN STUIVENBERG, Herman H., NL-1381 CP Weesp (NL); SLIEDREGT, Leonardus A.J.M., NL-1381 CP Weesp (NL)
(74) Representative: Aerts, Robert Jaap
(86) International application number: PCT/EP2004/052239
(87) International publication number: WO 2005/028456

(56) References cited:
- EP-A- 0 169 502
- EP-A- 0 199 968
- EP-A- 0 506 194
- WO-A-01/27094
- WO-A-03/007887
- WO-A-03/078413
- FR-A- 2 250 527
- FR-A- 2 261 756
- OHKUBO M ET AL: "Studies on cerebral protective agents. VIII. Synthesis of 2-aminothiazoles and 2-thiazolecarboxamides with anti-anoxic activity" CHEMICAL AND PHARMACEUTICAL BULLETIN, vol. 43, no. 9, September 1995 (1995-09), pages 1497-1504, XP002313044
- DATABASE CAPLUS CHEMICAL ABSTRACTS SERVICE, COLUMBUS, OHIO, US; XP002313045 retrieved from STN Database accession no. 1995:248274 & WO 94/20476 A (FUJISAWA PHARMACEUTICAL CO., LTD.) 15 September 1994 (1994-09-15)
- KULKARNI R A ET AL: "2-Benzoyl-4-substituted-phenyl- 5-phenylthiazoles" JOURNAL OF THE INDIAN CHEMICAL SOCIETY, vol. 65, June 1988 (1988-06), pages 427-428, XP008027244
- FALLAB: "Über Thiazolyl-(5)-carbinol" HELVETICA CHIMICA ACTA, vol. 35, 1952, pages 215-216,

## Description

The present invention relates to a group of thiazole derivatives, to methods for the preparation of these compounds, to pharmaceutical compositions containing at least one these compounds as active ingredient, as well to the use of these compositions for the treatment of psychiatric and neurological disorders and other diseases involving cannabinoid CB neurotransmission. The thiazole derivatives of the invention are either cannabinoid (CB) receptor antagonists, CB receptor agonists, CB receptor inverse agonists or CB receptor partial agonists. The thiazole derivatives of the invention bind either on the CB₁ receptor or on the CB₂ receptor or on both the CB₁ and CB₂ receptor.

The invention relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the invention specific compounds disclosed herein are used for the manufacture of a medicament.

Thiazoles have been claimed in WO0127094 as triglyceride inhibitors. WO0426863 describes thiazole derivatives as transforming growth factor (TgF) inhibitors. 4,5-Diarylthiazole derivatives have been described in EP 388909 and EP 377457 as 5-lipoxygenase inhibitors for the treatment of thrombosis, hypertension, allergy and inflammation. The exemplified structures therein all contain two phenyl rings which are p-substituted with a methoxy, fluoro, methylthio or methylsulfinyl group. WO 9603392 describes sulfonylaryl-arylthiazoles for inflammation and pain, arthritis or fever as inflammation-associated disorders. JP 05345772 relates to 4,5-diarylthiazoles as acetyl cholinesterase inhibitors, and JP 04154773 describes 4,5-diarylthiazoles having analgesic, anti-inflammatory and antipyretic action.
Thiazoles have not been disclosed as cannabinoid receptor ligands. As such, compounds most closely related to those of the present invention are the imidazole derivatives described in WO 03/007887.

It has now surprisingly been found that the thiazole derivatives of the formula (I), prodrugs thereof and salts thereof 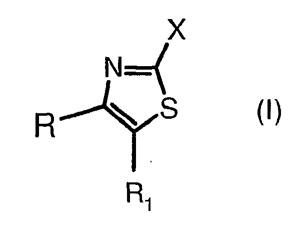 wherein
- R and R₁ are the same or different and represent phenyl or pyridinyl, optionally substituted with 1-3 substituents Y, wherein Y represents a substituent from the group methyl, ethyl, propyl, methoxy, ethoxy, hydroxy, hydroxymethyl, hydroxyethyl, chloro, iodo, bromo, fluoro, trifluoromethyl, trifluoromethoxy, methylsulfonyl, methylsulfanyl, trifluoromethylsulfonyl, phenyl or cyano, with the proviso that X does not represent the subgroup (ii),
   or one of the moieties R and R₁ represents a phenyl or pyridinyl group, optionally substituted with 1-3 substituents Y, wherein Y has the abovementioned meaning and the other moiety represents a hydrogen atom or a C₁₋₈ branched or linear alkyl group, G₃₋₈ branched or linear heteroalkyl group containing one heteroatom from the group (N, O, S), a C₃₋₇ cycloalkyl group, C₃₋₇-cycloalkyl-C₁₋₃-alkyl group, C₃₋₇-heterocycloalkyl-C₁₋₃-alkyl group which groups may be substituted with a hydroxy, methoxy, methyl, trifluoromethylsulfonyl or trifluoromethyl group or a fluoro atom and which C₃₋₇-heterocycloalkyl-C₁₋₃-alkyl group contains one or two heteroatoms from the group (O, N, S), or said other moiety represents a benzyl group optionally substituted on its phenyl ring with 1-3 substituents Y, wherein Y has the abovementioned meaning,
   X represents one of the subgroups (i) or (ii), 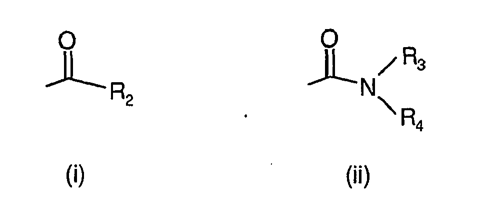 wherein
- R₂ represents a C₁₋₈ branched or linear alkyl group, C₃₋₇ cycloalkyl group, C₃₋₇-cycloalkyl-C₁₋₂-alkyl group, C₃₋₇-heterocycloalkyl-C₁₋₂-alkyl group which groups may be substituted with a hydroxy, methyl or trifluoromethyl group or a fluoro atom and which C₃₋₇-heterocycloalkyl-C₁₋₂-alkyl group contains one or two heteroatoms from the group (O, N, S), or R₂ represents a phenyl, benzyl, phenethyl or phenylpropylgroup which may be substituted on their phenyl ring with with 1-3 substituents Y, wherein Y has the abovementioned meaning, or R₂ represents a pyridyl, thienyl or naphtyl group, which napthyl group may be substituted with a halogen atom, a methyl group or a methoxy or trifluoromethyl group, with the proviso that when R₂ represents phenyl, R is not a 4-chlorophenyl group, and excluding 2-benzoyl-4,5-diphenylthiazole, 2-benzoyl-4-p-methylphenyl-5-phenylthiazole, 2-benzoyl-4-p-chlorophenyl-5-phenylthiazole and 2-benzoyl-4-p-methoxyphenyl-5-phenylthiazole,
   - R₃ represents a hydrogen atom or a branched or linear C₁₋₃ alkyl group,
   - R₄ represents hydrogen, a branched or linear C₁₋₁₀ alkyl or C₃₋₈-cycloalkyl-C₁₋₂-alkyl group, branched or linear C₁₋₁₀ alkoxy, C₃₋₈ cycloalkyl, C₅₋₁₀ bicycloalkyl, C₅₋₁₀-bicycloalkyl-C₁₋₂-alkyl, C₆₋₁₀ tricycloalkyl, C₆₋₁₀ tricycloalkyl-methyl, branched or linear C₃₋₁₀ alkenyl, C₅₋₈ cycloalkenyl, which groups may contain one or more heteroatoms from the group (O, N, S) and which groups may be substituted with a hydroxy group, 1-3 methyl groups, an ethyl group or 1-3 fluoro atoms, or R₄ represents a phenyl, phenylamino, phenoxy, benzyl, phenethyl or phenylpropyl group, optionally substituted on their phenyl ring with 1-3 substituents Y, wherein Y has the abovementioned meaning, or R₄ represents a pyridyl or thienyl group, or R₄ represents a group NR₅R₆ wherein
      R₅ and R₆ - together with the nitrogen atom to which they are attached -form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, hydroxy or trifluoromethyl group or a fluoro atom, or
      R₃ and R₄ - together with the nitrogen atom to which they are attached - form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, amino, hydroxy, methoxy, cyano or trifluoromethyl group or a fluoro or chloro atom,
   are modulators of the cannabinoid CB receptor.

To the invention belong all compounds having formula (I), racemates, mixtures of diastereomers and the individual steroisomers. Thus compounds in which the substituents on potentially asymmetrical carbon atoms are in either the R-configuration or the S-configuration belong to the invention.

Due to the CB receptor activity the compounds according to the invention are suitable for use in the treatment of psychiatric disorders such as psychosis, anxiety, depression, attention deficits, memory disorders, cognitive disorders, appetite disorders, obesity, addiction, appetence, drug dependence, neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, spinal cord injury, neuroinflammatory disorders, plaque sclerosis, viral encephalitis, demyelinisation related disorders, as well as for the treatment of pain disorders, including neuropathic pain disorders, septic shock, glaucoma, cancer, diabetes, emesis, nausea, asthma, respiratory diseases, gastrointestinal disorders, sexual disorders, gastric ulcers, diarrhoea and cardiovascular disorders.

### PHARMACOLOGICAL METHODS

### In vitro affinity for human cannabinoid CB₁ receptors

The affinity of the compounds of the invention for cannabinoid CB₁ receptors can be determined using membrane preparations of Chinese hamster ovary (CHO) cells in which the human cannabinoid CB₁ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro affinity for human cannabinoid CB₂ receptors

The affinity of the compounds of the invention for cannabinoid CB₂ receptors can be determined using membrane preparations of Chinese hamster ovary (CHO) cells in which the human cannabinoid CB₂ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro antagonism on human cannabinoid CB₁ receptors

In vitro CB₁ receptor antagonism can be assessed with the human CB₁ receptor cloned in Chinese hamster ovary (CHO) cells. CHO cells are grown in a Dulbecco's Modified Eagle's medium (DMEM) culture medium, supplemented with 10% heat-inactivated fetal calf serum. Medium is aspirated and replaced by DMEM, without fetal calf serum, but containing [³H]-arachidonic acid and incubated overnight in a cell culture stove (5% CO₂/95% air; 37 °C; water-saturated atmosphere). During this period [³H]-arachidonic acid is incorporated in membrane phospholipids. On the test day, medium is aspirated and cells are washed three times using 0.5 mL DMEM, containing 0.2% bovine serum albumin (BSA). Stimulation of the CB₁ receptor by WIN 55,212-2 leads to activation of PLA₂ followed by release of [³H]-arachidonic acid into the medium. This WIN 55,212-2-induced release is concentration-dependently antagonized by CB₁ receptor antagonists. The CB₁ antagonistic potencies of the test compounds are expressed as pA₂ values.

### In vivo antagonism on human cannabinoid CB₁ receptors

In vivo CB₁ antagonism can be assessed with the CP-55,940-induced hypotension test in rat. Male normotensive rats (225-300 g; Harlan, Horst, The Netherlands) are anaesthetized with pentobarbital (80 mg/kg ip). Blood pressure is measured, via a cannula inserted into the left carotid artery, by means of a Spectramed DTX-plus pressure transducer (Spectramed B.V., Bilthoven, The Netherlands). After amplification by a Nihon Kohden Carrier Amplifier (Type AP-621 G; Nihon Kohden B.V., Amsterdam, The Netherlands), the blood pressure signal is registered on a personal computer (Compaq Deskpro 386s), by means of a Po-Ne-Mah data-acquisition program (Po-Ne-Mah Inc., Storrs, USA). Heart rate is derived from the pulsatile pressure signal. All compounds are administered orally as a microsuspension in 1% methylcellulose 30 minutes before induction of the anesthesia which is 60 minutes prior to administration of the CB₁ receptor agonist CP-55,940. The injection volume is 10 mL kg⁻¹. After haemodynamic stabilization the CB₁ receptor agonist CP-55,940 (0.1 mg kg⁻¹ i.v.) is administered and the hypotensive effect established. (Wagner, J.A. et al., Hemodynamic effects of cannabinoids: coronary and cerebral vasodilation mediated by cannabinoid CB1 receptors. Eur. J. Pharmacol. 2001, 423,203-210).
This hypotension test can also be used to assess CB₁ receptor agonistic effects of the compounds. Such CB₁ agonistic effects on blood pressure may be counteracted by a selective CB₁ receptor antagonist such as rimonabant.

Cannabinoid receptor agonistic or partial agonistic activity of compounds of the invention can be determined according to published methods, such as assessment of in vivo cannabimimetic effects (Wiley, J. L. et al., J Pharmacol. Exp. Ther. 2001, 296, 1013).

The compounds of the invention can be brought into forms suitable for administration by means of usual processes using auxiliary substances and/or liquid or solid carrier materials.

Compounds of the present invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art.

In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### GENERAL ASPECTS OF SYNTHESES

Thiazole derivatives can be obtained according to methods known, for example.
a) Organic Reactions, Vol. VI, (1951), p. 367-409, Ed. R. Adams, John Wiley and Sons Inc., New York
b) J. S. Carter et al., Bioorg. Med. Chem. Lett. (1999), 9, 1167-1170
c) T. T. Sakai et al., Bioorg. Med. Chem. (1999), 7, 1559-1566
d) A. Tanaka et al., J. Med. Chem. (1994), 37,1189-1199
e) J. J. Talley et al., WO 9603392: Chem. Abstr. 125, 33628
f) V. Cecchetti et al., Bioorg. Med. Chem. (1994), 2, 799-806
g) T. Eicher et al., The Chemistry of Heterocycles, (1995) p. 149-155, Georg Thieme Verlag, Stuttgart, 1995, ISBN 313-100511-4, and references cited therein
h) Gilchrist, T.L., Heterocyclic Chemistry, 3th Ed. 1997, p. 319-327, Longman, UK, ISBN 0-582-27843-0.

### Alfahaloketones can be obtained by halogenation of the corresponding ketone.

The reaction of alfa-halo carbonyl compounds and thioamide can produce a wide range of thiazole derivatives. More in particular, condensation of alfa-bromoketones with ethyl thiooxamate provides (2-ethoxy-carbonyl)thiazoles of general formula (II). 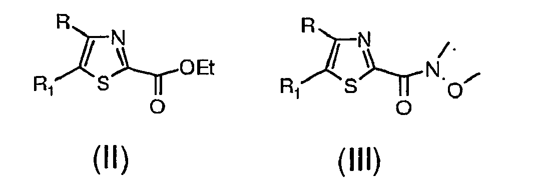 Compounds of formula (II) can be converted to the corresponding Nmethoxy-N-methylamide (III) and subsequently reacted with an alkyllithium or aryl lithium reagent to give a compound of general formula (I), wherein X represents subgroup (i).

Compounds of general formula (II) can be amidated with an amine of general formula R₃R₄NH into a compound of general formula (I) wherein X represents subgroup (ii). Such amidations can be catalyzed by (CH₃)₃Al. (For more information on aluminum-mediated conversion of esters to amides, see: J. I. Levin, E. Turos, S. M. Weinreb, Synth. Commun. (1982), 12, 989-993.)
Alternatively, a compound having formula (II) is converted into the corresponding carboxylic acid and subsequently reacted with a so-called halogenating agent such as for example thionyl chloride (SOCl₂). This reaction gives the corresponding carbonyl chloride that is subsequently reacted with a compound having formula R₃R₄NH wherein R₃ and R₄ have the meanings as described above.
Alternatively, the ester group in (II) can be converted to the corresponding carboxylic acid. This carboxylic acid can be reacted with a compound having formula R₃R₄NH wherein R₃ and R₄ have the meanings as described hereinabove via activating and coupling methods such as formation of an active ester, or in the presence of a so-called coupling reagent, such as for example, DCC, HBTU, HOAT (N-hydroxy-7-azabenzotriazole), BOP, CIP (2-chloro-1,3-dimethylimidazolinium hexafluorophosphate), PyAOP (7-azabenzotriazol-1-yloxytris(pyrrolidino)-phosphonium hexafluorophosphate) and the like. (For more information on activating and coupling methods see a) M. Bodanszky, A. Bodanszky: The Practice of Peptide Synthesis, Springer-Verlag, New York, 1994; ISBN: 0-387-57505-7; b) K. Akaji et al., Tetrahedron Lett. (1994), 35, 3315-3318; c) F. Albericio et al., Tetrahedron Lett. (1997), 38, 4853-4856).

According to these procedures the following compounds can be prepared. They are intended to further illustrate the invention in more detail, and therefore are not deemed to restrict the scope of the invention in any way.

### SYNTHESES OF SPECIFIC EXAMPLES

¹H NMR spectra were recorded on a Varian UN400 instrument (400 MHz) with tetramethylsilane as an internal standard. Chemical shifts are given in ppm (d?scale) downfield from tetramethylsilane. Coupling constants *(J)* are expressed in Hz. Thin-layer chromatography was performed on Merck pre-coated 60 F₂₅₄ plates, and spots were visualised with UV light. Flash chromatography was performed using silica gel 60 (0.040-0.063 mm, Merck). Column chromatography was performed using silica gel 60 (0.063-0.200 mm, Merck). Melting points were recorded on a Büchi B545 melting point apparatus and are uncorrected.

### Example 1

Part A: To a solution of 1-(2,4-dichlorophenyl)-2-phenylethanone (54.35 gram, 0.205 mol) in benzene (220 mL) is slowly added bromine (10.6 mL, 0.205 mol) and the resulting solution is stirred at room temperature for 1 hour. Aqueous (5 %) NaHCO₃ solution is slowly added. The organic layer is separated, dried over MgSO₄, filtered and evaporated *in vacuo* to give crude 2-bromo-1-(2,4-didhlorophenyl)-2-phenylethanone (69.4 g, 98 % yield) as an oil. ¹H-NMR (400 MHz, CDCl₃): d 6.20 (s, 1H), 7.26 (dd, J = 8 and 2 Hz, 1H), 7.31-7.50 (m, 7H).
**Part B**; 2-Bromo-1-(2,4-dichlorophenyl)-2-phenylethanone (25.83 gram, 0.075 mol) and ethyl thiooxamate (15.0 gram, 0.112 mol) are dissolved in absolute ethanol (200 mL). The resulting mixture is heated at reflux temperature for 16 hours. After evaporation *in vacuo* the crude material is dissolved in a mixture of water and dichloromethane. The dichloromethane layer is separated and the water layer is extracted three times with dichloromethane. The collected organic layers are dried (MgSO₄), filtered and concentrated. The resulting material is purified by column chromatography (silica gel / dichloromethane) to give ethyl 4(2,4-dichlorophenyl)-5-phenylthiazole-2-carboxylate (10.5 gram, 37 % yield). ¹H-NMR (400 MHz, CDCl₃): d P.46 (t, J = 7 Hz, 3H), 4.53 (q, J = 7 Hz, 2H), 7.24-7.38 (m, 7H), 7.43 (d, J = 2 Hz, 1H).
**Part C**: To a solution of ethyl 4(2,4-dichlorophenyl)-5-phenylthiazole-2-carboxylate (10.5 g, 0.028 mol) in methanol (170 ml) is slowly added a solution of KOH (8.9 g, 0.0896 mol) in water (170 ml). The resulting solution is heated at 90 °C for 2 hours and cooled to room temperature. A mixture of concentrated HCl and ice is added. The formed precipitate is collected, washed with water and diethyl ether and dried to give 4-(2,4-dichlorophenyl)-5-phenylthiazole-2-carboxylic acid (8.99 gram, 92 % yield). Melting point: 105 °C.
**Part D**: To a magnetically stirred suspension of 4-(2,4-dichlorophenyl)-5-phenylthiazole-2-carboxylic acid (4.2 g, 0.012 mol) in anhydrous dichloromethane (170 ml) is successively added 7-aza-1-hydroxybenzotriazole (HOAT) (4.083 gram, 0.030 mol), 7-azabenzotriazol-1-yloxytris(pyrrolidino)phosphonium hexafluorophosphate (PyAOP) (15.64 gram, 0.03 mol), diisopropylethylamine (6.26 ml, 0.036 mol) and N-methoxy-N-methylamine.HCl (2.925 gram, 0.030 mol) and the resulting solution is stirred for 16 hours at room temperature. A 5 % aqueous NaHCO₃ solution is slowly added and the resulting mixture is extracted (3x) with dichloromethane. The collected organic layers are dried (MgSO₄), filtered and concentrated to give a crude oil (18.9 gram). Purification by flash chromatography (silica gel / ethyl acetate/petroleum ether = 1/1) gives N-methyl-N-methoxy-4-(2,4-dichlorophenyl)-5-phenylthiazole-2-carboxamide (4.0 gram, 85 % yield). ¹H-NMR (400 MHz, CDCl₃): d 3.60 (br s, 3H), 3.90 (s, 3H), 7.21-7.33 (m, 7H), 7.45 (d, J = 2 Hz, 1H).
   Analogously was prepared: N-methyl- N-methoxy-4-(2-chlorophenyl)-5-phenylthiazole-2-carboxamide. ¹H-NMR (400 MHz, CDCl₃): d 3.62 (br s, 3H), 3.90 (s, 3H), 7.22-7.45 (m, 9H).
**Part E**: To a cooled (- 70 °C) and stirred solution of N-methyl-N-methoxy-4-(2,4-dichlorophenyl)-5-phenylthiazole-2-carboxamide (2.0 gram, 0.005 mol) in THF (20 ml) under N₂ is added n-BuLi (3.13 ml, 1.6 M solution in hexane, 0.005 mol). After stirring for 30 minutes the solution is allowed to attain room temperature and stirred for 16 hours. Aqueous HCl (20 ml, 1 N) is added and the resulting mixture is extracted with diethyl ether. The diethyl ether layers are washed with water (2x), dried (MgSO₄), filtered and concentrated to give a crude oil (2.03 gram). Purification by flash chromatography (silica gel / dichloromethane) gives 1-[4-(2,4-dichlorophenyl)-5-phenylthiazol-2-yl]pentan-1-one (0.6 gram, 31 % yield). ¹H-NMR (400 MHz, CDCl₃): d 0.95 (t, J = 7 Hz, 3H), 1.38-1.48 (m, 2H), 1.72-1.80 (m, 2H), 3.16 (t, J ∼ 7 Hz, 2H), 7.20-7.35 (m, 7H), 7.46 (d, J = 2 Hz, 1H).

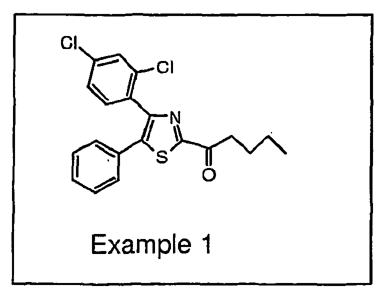

Analogously were prepared:

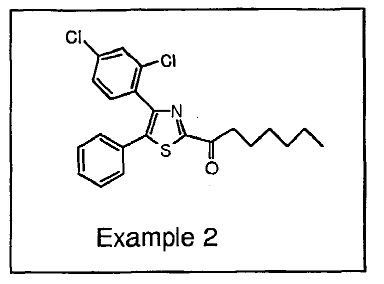

### Example 2: ¹H-NMR (400 MHz, CDCl₃): d 0.93 (t, J = 7 Hz, 3H), 1.26-1.44 (m, 6H), 1.73-1.82 (m, 2H), 3.15 (t, J ∼ 7 Hz, 2H), 7.21-7.34 (m, 7H), 7.47 (d, J = 2 Hz, 1H).

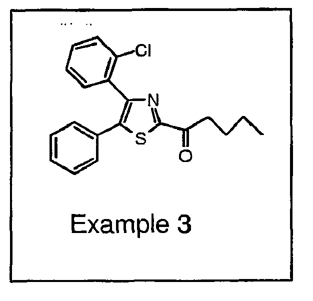

### Example 3: Melting point: 78-80 °C.

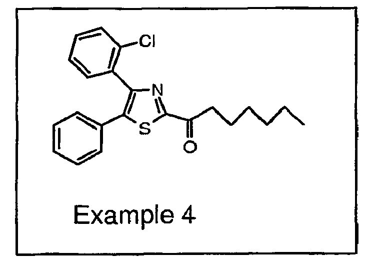

### Example 4: ¹H-NMR (400 MHz, CDCl₃): d 0.93 (t, J = 7 Hz, 3H), 1.25-1.44 (m, 6H), 1.73-1.82 (m, 2H), 3.17 (t, J ∼ 7 Hz, 2H), 7.22-7.40 (m, 8H), 7.47 (dd, J = 8 and 2 Hz, 1H).

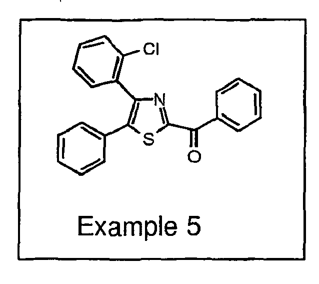

### Example 5: Melting point: 131-132 °C.

### Example 6

**Part A**: To a magnetically stirred solution of 1-phenylheptan-1-one (23.7 gram, 0.125 mol) in benzene (160 mL) is slowly added bromine (7.0 mL, 0.125 mol) and he resulting solution is reacted at room temperature for 1 hour. Aqueous (5 %) NaHCO₃ solution is slowly added, followed by dichloromethane. The organic layer is separated, dried over MgSO₄, filtered and evaporated *in vacuo* to give crude 2 bromo-1-phenylheptan-1-one (41.8 g, quantitative yield) as an oil. ¹H-NMR (400 MHz; CDCl₃): d?0.90 (t, J = 7 Hz, 3H), 1.28-1.78 (m, 6H), 2.04-2.25 (m, 2H), 5.11-5.16 (m, 1H), 7.42-7.62 (m, 3H), 8.00-8.04 (m, 2H).
**Part B**; 2-Bromo-1-phenylheptan-1-one (20.17 gram, 0.075 mol) and ethyl thiooxamate (15.0 gram, 0.112 mol) are dissolved in absolute ethanol (200 mL). The resulting mixture is heated at reflux temperature for 16 hours. After evaporation *in vacuo* the crude material is dissolved in a mixture of water and dichloromethane. The dichloromethane layer is separated and the water layer is extracted three times with dichloromethane. The collected organic layers are dried (MgSO₄) filtered and concentrated. The resulting material is purified by column chromatography (silica gel / dichloromethane/petroleum ether = 1/1) to give ethyl 5-(n-pentyl)-4-phenylthiazole-2-carboxylate (12.09 gram, 53 % yield) as an oil which slowly solidified. Melting point: 51-52 °C.
**Part C**: To a magnetically stirred solution of ethyl 5(n-pentyl)-4-phenylthiazole-2-carboxylate (12.09 g, 0.039 mol) in methanol (240 ml) is slowly added a solution of KOH (8.9 g) in water (240 ml). The resulting solution is heated at reflux temperature for 2 hours and subsequently cooled to room temperature. A mixture of concentrated HCl and ice is added. The formed precipitate is collected, successively washed with water and cold diethyl ether and dried to give 5-(n-pentyl)-4-phenylthiazole-2-carboxylic acid (3.54 gram, 32 % yield). ¹H-NMR (400 MHz, CDCl₃): d?0.87 (t, J = 7 Hz, 3H), 1.28-1.40 (m, 4H), 1.66-1.76 (m, 2H), 2.93-3.00 (m, 2H), 4.00 (br s, 1H), 7.35-7.46 (m, 3H), 7.58-7.64 (m, 2H).
**Part D:** To a magnetically stirred suspension of 5-(n-pentyl)-4-phenylthiazole-2-carboxylic acid (1.18 g, 0.0043 mol) in anhydrous dichloromethane (35 ml) is successively added 7-aza-1-hydroxybenzotriazole (HOAT) (1.46 gram, 0.0107 mol), 7-azabenzotriazol-1-yloxytris(pyrrolidino)phosphonium hexafluorophos- phate (PyAOP) (5.59 gram, 0.0107 mol), diisopropylethylamine (2.24 ml, 0.0129 mol) and aniline (0.98 ml, 0.0107 mol) and the resulting solution is stirred for 16 hours at room temperature. The resulting mixture is concentrated and purified by flash chromatography (silica gel / dichloromethane) to give N-phenyl-5-(n-pentyl)-4-phenylthiazole-2-carboxamide (0.89 gram, 59 % yield). ¹H-NMR (400 MHz, CDCl₃): d 0.98 (t, J = 7 Hz, 3H), 1.26-1.41 (m, 4H), 1.68-1.78 (m, 2H), 2.97 (t, J = 7 Hz, 2H), 7.12-7.18 (m, 1H), 7.34-7.52 (m, 5H),
   7.60-7.64 (m, 2H), 7.69-7.74 (m, 2H), 9.10 (br s 1H).

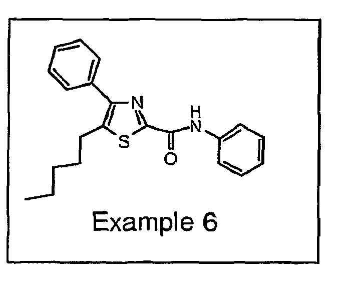

Analogously were prepared: 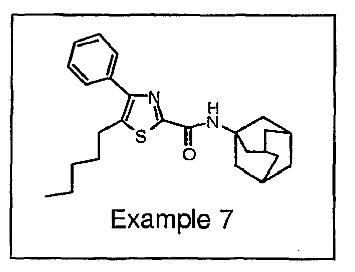 from 5-(n-pentyl)-4-phenylthiazole-2-carboxylic acid and 1-aminoadamantane.

### Example 7: Melting point: 90-92 °C.

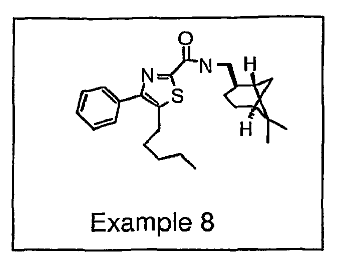

from 5-(n-pentyl)-4-phenylthiazole-2-carboxylic acid and cis-myrtanylamine (CAS 38235-68-6)

### Example 8: ¹H-NMR (400 MHz, CDCl₃): d 0.89 (t, J = 7 Hz, 3H), 1.08 (s, 3H), 1.20 (s, 3H), 1.26-1.38 (m, 4H), 1.50-1.62 (m, 1H), 1.66-1.74 (m, 2H), 1.82-2.04 (m, 5H), 2.28-2.40 (m, 2H), 2.94 (t, J = 7 Hz, 2H), 3.39-3.50 (m, 2H), 7.29 (br t, J ∼ 7 Hz, 1H), 7.37-7.60 (m, 5H).

### Example 9

**Part A**: 1-Bromo-1-phenylheptan-2-one (19.98 gram, 0.074 mol) and ethyl thiooxamate (15.0 gram, 0.112 mol) are dissolved in absolute ethanol (200 mL). The resulting mixture is heated at reflux temperature for 2 hours. After evaporation *in vacuo* the crude material is dissolved in a mixture of water and dichloromethane. The dichloromethane layer is separated and the water layer is extracted three times with dichloromethane. The collected organic layers are dried (MgSO₄), filtered and concentrated. The resulting material is purified by column chromatography (silica gel / dichloromethane/petroleum ether = 1/1) to give ethyl 4-(n-pentyl)-5-phenylthiazoie-2-carboxylate (5.24 gram, 23 % yield) as an oil. ¹H-NMR (400 MHz, CDCl₃): d 0.89 (t, J = 7 Hz, 3H), 1.24-1.32 (m, 4H), 1.44 (t, J = 7 Hz, 3H), 1.70-1.78 (m, 2H), 2.81-2.87 (m, 2H), 4.48 (q, J = 7 Hz, 2H), 7.40-7.48 (m, 5H).
   Analogously were prepared:
   Ethyl 4-benzyl-5-phenylthiazole-2-carboxylate as an oil.
   Ethyl 5-(n-pentyl)-4-(2,4-dichorophenyl)thiazole-2-carboxylate. Melting point: 92-93 °C.
**Part B**: To a magnetically stirred solution of 1-aminoadamantane (1.607 gram, 0.0086 mol) in anhydrous dichloromethane (10 ml) is added Al(CH₃)₃ (4.3 mL, 2M solution in hexane, 0.0086 mol) and the resulting solution is reacted at room temperature for 10 minutes. Aqueous (5 %) NaHCO₃ solution is slowly added. Extraction with dichloromethane, drying over MgSO₄, filtration and concentration *in vacuo,* followed by column chromatography (silica gel / dichloromethane) gives N-(adamant-1-yl)-4-(n-pentyl)-5-phenylthiazole-2-carboxamide (1.17 g, 72 % yield). ¹H-NMR (400 MHz, CDCl₃): d ?0.88 (t, J = 7 Hz, 3H), 1.26-1.34 (m, 4H), 1.68-1.78 (m, 8H), 2.08-2.18 (m, 9H), 2.71-2.76 (m, 2H), 7.02 (br s, 1H), 7.36-7.45 (m, 5H).

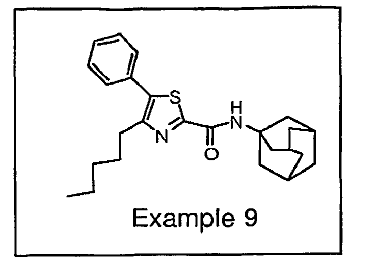

Analogously were prepared: 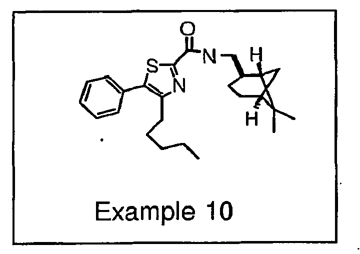 from ethyl 4-(n-pentyl)-5-phenylthiazole-2-carboxylate and cis-myrtanylamine (GAS 38235-68-6)

### Example 10: ¹H-NMR (400 MHz, CDCl₃): d 0.89 (t, J = 7 Hz, 3H), 1.10 (s, 3H), 1.22 (s, 3H), 1.25-1.34 (m, 4H), 1.54-1.78 (m, 3H), 1.84-2.06 (m, 5H), 2.31-2.42 (m, 2H), 2.72-2.78 (m, 2H), 3.44-3.50 (m, 2H), 7.24-7.28 (m, 1H), 7.37-7.46 (m, 5H).

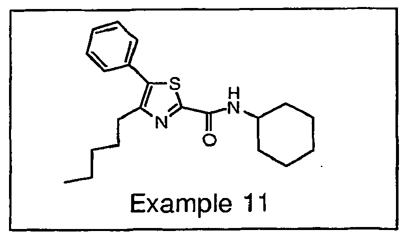

from ethyl 4-(n-pentyl)-5-phenylthiazole-2-carboxylate and cyclohexylamine

### Example 11: Melting point: 84-85 °C.

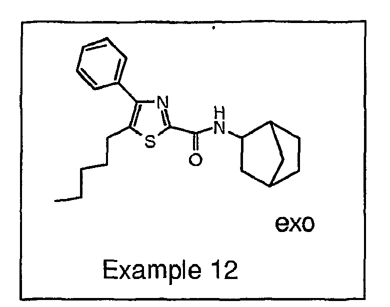

from ethyl 5-(n-pentyl)-4-phenylthiazole-2-carboxylate and exo-2-aminobicyclo[2.2.1]heptane

### Example 12: Melting point: 64-65 °C.

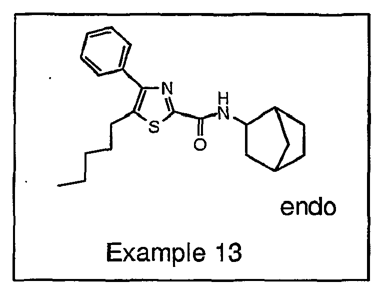

from ethyl 5-(n-pentyl)-4-phenylthiazole-2-carboxylate and endo-2-aminobicyclo[2.2.1]heptane

### Example 13: Melting point: 80-82 °C.

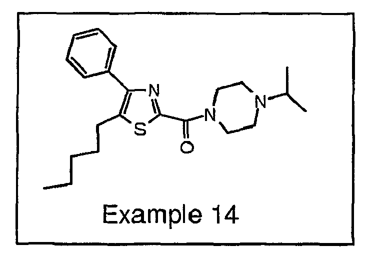

from ethyl 5-(n-pentyl)-4-phenylthiazole-2-carboxylate and 4-isopropylpiperazine

### Example 14: Melting point: 84-85 °C.

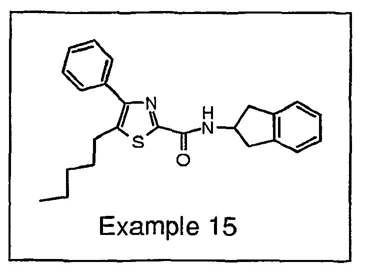

from ethyl 5-(n-pentyl)-4-phenylthiazole-2-carboxylate and indan-2-ylamine

### Example 15: ¹H-NMR (400 MHz, CDCl₃): d 0.85 (t, J = 7 Hz, 3H), 1.26-1.38 (m, 4H), 1.65-1.75 (m, 2H), 2.90-3.01 (m, 4H), 3.36-3.44 (m, 2H), 4.86-4.96 (m, 1H), 7.15-7.27 (m, 4H), 7.35-7.47 (m, 4H), 7.52-7.56 (m, 2H).

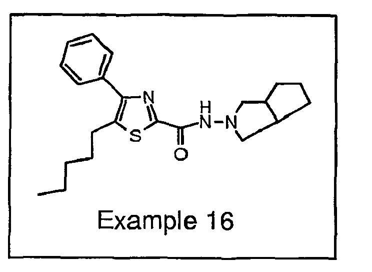

from ethyl 5-(n-pentyl)-4-phenylthiazole-2-carboxylate and 3-amino-3-azabicyclo[3.3.0]octane

### Example 16: Melting point: 86-87 °C.

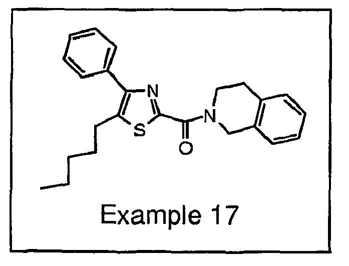

from ethyl 5-(n-pentyl)-4-phenylthiazole-2-carboxylate and 1,2,3,4-tetrahydroisoquinoline.

### Example 17: Melting point: 50-51 °C.

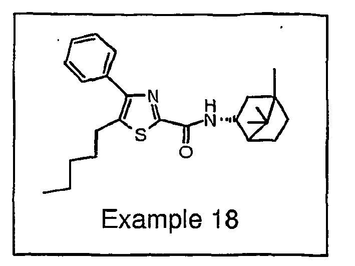

from ethyl 5-(n-pentyl)-4-phenylthiazole-2-carboxylate and R-(+)-bornylamine (CAS 32511-34-5).

### Example 18: ¹H-NMR (400 MHz, CDCl₃): d 0.90-1.02 (m, 13H), 1.22-1.47 (m, 6H), 1.60-1.84 (m, 5H), 2.36-2.45 (m, 1H), 2.94 (t, J = 7 Hz, 2H), 4.36-4.44 (m, 1H), 7.32 (br d, J ∼ 7 Hz, 1H), 7.38-7.50 (m, 3H), 7.58-7.63 (m, 2H).

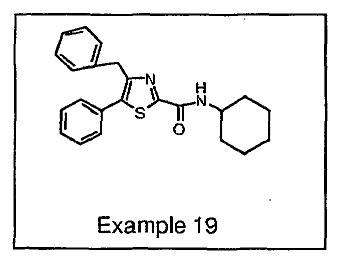

from ethyl 4-benzyl-5-phenylthiazole-2-carboxylate and cyclohexylamine

### Example 19: Melting point: 104-106 °C.

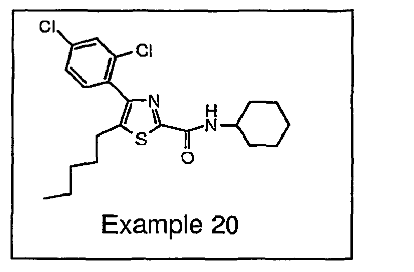

from ethyl 5-(n-pentyl)-4-(2,4-dichlorophenyl)thiazole-2-carboxylate and cyclohexylamine

### Example 20: ¹H-NMR (400 MHz, CDCl₃): d 0.85 (t, J = 7 Hz, 3H), 1.13-1.46 (m, 8H), 1.55-1.68 (m, 4H), 1.71-1.80 (m, 2H), 1.96-2.06 (m, 2H), 2.67 (t, J = 7 Hz, 2H), 3.86-3.98 (m, 1H), 7.05 (br d, J = 7 Hz, 1H), 7.26-7.37 (m, 2H), 7.53 (d, J = 2 Hz, 1H).

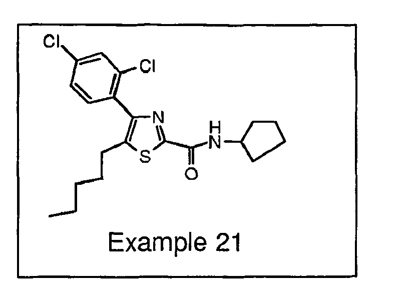

from ethyl 5-(n-pentyl)-4-(2,4-dichlorophenyl)thiazole-2-carboxylate and cyclopentylamine

### Example 21: ¹H-NMR (400 MHz, CDCl₃): d 0.85 (t, J = 7 Hz, 3H), 1.20-1.30 (m, 4H), 1.48-1.77 (m, 8H), 2.01-2.10 (m, 2H), 2.67 (t, J = 7 Hz, 2H), 4.31-4.41 (m, 1H), 7.09 (br d, J = 7 Hz, 1H), 7.25-7.37 (m, 2H), 7.53 (d, J = 2 Hz, 1H).

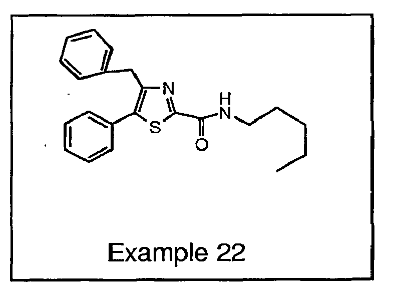

from ethyl 4-benzyl-5-phenylthiazole-2-carboxylate and n-pentyiamine

### Example 22: ¹H-NMR (400 MHz, CDCl₃): d 0.89 (t, J = 7 Hz, 3H), 1.33-1.40 (m, 4H), 1.59-1.67 (m, 2H), 3.40-3.47 (m, 2H), 4.16 (s, 2H), 7.15-7.32 (m, 6H), 7.39-7.42 (m, 5H).

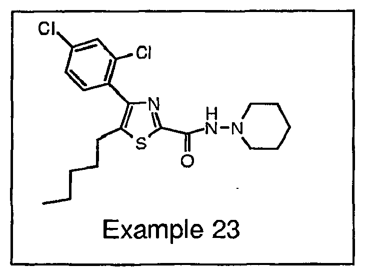

from ethyl 5-(n-pentyl)-4-(2,4-dichlorophenyl)thiazole-2-carboxylate and 1-aminopiperidine

### Example 23: ¹H-NMR (400 MHz, CDCl₃): d 0.85 (t, J = 7 Hz, 3H), 1.20-1.28 (m, 4H), 1.39-1.46 (m, 2H), 1.56-1.64 (m, 2H), 1.71-1.79 (m, 4H), 2.66 (t, J = 7 Hz, 2H), 2.82-2.88 (m, 4H), 7.29 (d, J = 8 Hz, 1H), 7.35 (dd, J = 8 and 2 Hz, 1H), 7.53 (d, J = 2 Hz, 1H), 7.88 (br s, 1H).

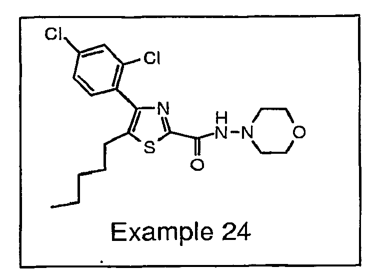

from ethyl 5-(n-pentyl)-4-(2,4-dichlorophenyl)thiazole-2-carboxylate and 4-aminomorpholine

### Example 24: ¹H-NMR (400 MHz, CDCl₃): d 0.85 (t, J = 7 Hz, 3H), 1.21-1.29 (m, 4H), 1.57-1.66 (m, 2H), 2.67 (t, J = 7 Hz, 2H), 2.93-2.98 (m, 4H), 3.82-3.88 (m, 4H), 7.29 (d, J = 8 Hz, 1H), 7.35 (dd, J = 8 and 2 Hz, 1H), 7.54 (d, J = 2 Hz, 1H), 7.95 (br s, 1H).

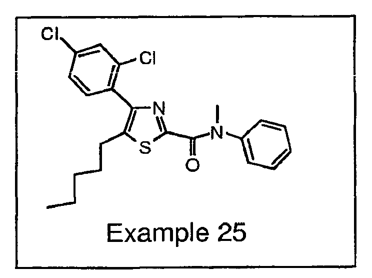

from ethyl 5-(n-pentyl)-4-(2,4-dichlorophenyl)thiazole-2-carboxylate and N-methylaniline

### Example 25: ¹H-NMR (400 MHz, CDCl₃): δ 0.80 (br t, J ∼ 7 Hz, 3H), 1.14-1.28 (m, 4H), 1.50-1.62 (m, 2H), 2.56-2.66 (m, 2H), 3.56 (br s, 3H), 6.80-7.45 (m, 8H).

### PHARMACOLOGICAL TESTRESULTS

Cannabinoid receptor affinity data obtained according to the protocols given above are shown in the table below.

| | Receptor affinity | |
|---|---|---|
| | Human CB₁ | Human CB₂ |
| Compound | pKᵢ-value | pKᵢ-value |
| | | |
| **Example 1** | **6.6** | |
| **Example 4** | **6.8** | |
| **Example 7** | **7.3** | |
| **Example 8** | **6.8** | |
| **Example 9** | **7.8** | **8.1** |
| **Example 10** | **6.9** | **8.1** |
| **Example 11** | **6.6** | **7.7** |

## Claims

1. Use of a compound of formula (I) 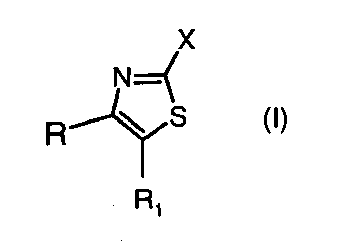 wherein
- R and R₁ are the same or different and represent phenyl or pyridinyl, optionally substituted with 1-3 substituents Y, wherein Y represents a substituent from the group methyl, ethyl, propyl, methoxy, ethoxy, hydroxy, hydroxymethyl, hydroxyethyl, chloro, iodo, bromo, fluoro, trifluoromethyl, trifluoromethoxy, methylsulfonyl, methylsulfanyl, trifluoromethylsulfonyl, phenyl or cyano, with the proviso that X does not represent the subgroup (ii),
or one of the moieties R and R₁ represents a phenyl or pyridinyl group, optionally substituted with 1-3 substituents Y, wherein Y has the abovementioned meaning and the other moiety represents a hydrogen atom or a C₁₋₈ branched or linear alkyl group, C₃₋₈ branched or linear heteroalkyl group containing one heteroatom from the group (N, O, S), a C₃₋₇ cycloalkyl group, C₃₋₇-cycloalkyl-C₁₋₃-alkyl group, C₃₋₇-heterocycloalkyl-C₁₋₃-alkyl group which groups may be substituted with a hydroxy, methoxy, methyl, trifluoromethylsulfonyl or trifluoromethyl group or a fluoro atom and which C₃₋₇-heterocycloalkyl-C₁₋₃-alkyl group contains one or two heteroatoms from the group (O, N, S), or said other moiety represents a benzyl group optionally substituted on its phenyl ring with 1-3 substituents Y, wherein Y has the abovementioned meaning,
X represents one of the subgroups (i) or (ii), 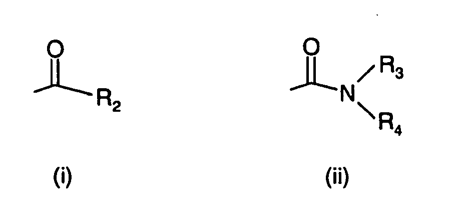 wherein
- R₂ represents a C₁₋₈ branched or linear alkyl group, C₃₋₇ cycloalkyl group, C₃₋₇-cycloalkyl-C₁₋₂-alkyl group, C₃₋₇-heterocycloalkyl-C₁₋₂-alkyl group which groups may be substituted with a hydroxy, methyl or trifluoromethyl group or a fluoro atom and which C₃₋₇-heterocydoalkyl-C₁₋₂-alkyl group contains one or two heteroatoms from the group (O, N, S), or R₂ represents a phenyl, benzyl, phenethyl or phenylpropylgroup which may be substituted on their phenyl ring with with 1-3 substituents Y, wherein Y has the abovementioned meaning, or R₂ represents a pyridyl, thienyl or naphtyl group, which napthyl group may be substituted with a halogen atom, a methyl group or a methoxy or trifluoromethyl group,
- R₃ represents a hydrogen atom or a branched or linear C₁₋₃ alkyl group,
- R₄ represents hydrogen, a branched or linear C₁₋₁₀ alkyl or C₃₋₈-cycloalkyl-C₁₋₂-alkyl group, branched or linear C₁₋₁₀ alkoxy, C₃₋₈ cycloalkyl, C₅₋₁₀ bicycloalkyl, C₅₋₁₀-bicycloalkyl-C₁₋₂-alkyl, C₆₋₁₀ tricycloalkyl, C₆₋₁₀ tricycloalkyl-methyl, branched or linear C₃₋₁₀ alkenyl, C₅₋₈ cycloalkenyl, which groups may contain one or more heteroatoms from the group (O, N, S) and which groups may be substituted with a hydroxy group, 1-3 methyl groups, an ethyl group or 1-3 fluoro atoms, or R₄ represents a phenyl, phenylamino, phenoxy, benzyl, phenethyl or phenylpropyl group, optionally substituted on their phenyl ring with 1-3 substituents Y, wherein Y has the abovementioned meaning, or R₄ represents a pyridyl or thienyl group, or R₄ represents a group NR₅R₆ wherein
R₅ and R₆ - together with the nitrogen atom to which they are attached -form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, hydroxy or trifluoromethyl group or a fluoro atom, or
R₃ and R₄ - together with the nitrogen atom to which they are attached - form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, amino, hydroxy, methoxy, cyano or trifluoromethyl group or a fluoro or chloro atom,
and pharmacologically acceptable salts thereof, for the preparation of a pharmaceutical composition for the treatment of psychosis, anxiety, depression, attention deficits, addiction, appetence, drug dependence, dystonia, muscle spasticity, tremor, Parkinson's disease, epilepsy, Huntington's disease, Tourette's syndrome, spinal cord injury, viral encephalitis, pain disorders, septic shock, glaucoma, cancer, emesis, nausea, sexual disorders and diarrhoea.

2. A compound of formula (I) 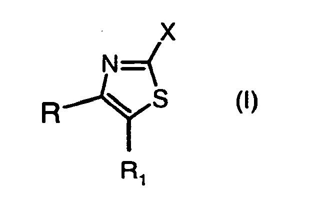 wherein
- R and R₁ are the same or different and represent phenyl, 3-pyridinyl or 4-pyridinyl, optionally substituted with 1-3 substituents Y, wherein Y represents a substituent from the group methyl, ethyl, propyl, methoxy, ethoxy, hydroxy, hydroxymethyl, hydroxyethyl, chloro, iodo, bromo, fluoro, trifluoromethyl, trifluoromethoxy, methylsulfonyl, methylsulfanyl, trifluoromethylsulfonyl, phenyl or cyano, with the proviso that X does not represent the subgroup (ii),
or one of the moieties R and R₁ represents a phenyl, 3-pyridinyl or 4-pyridinyl group, optionally substituted with 1-3 substituents Y, wherein Y has the abovementioned meaning and the other moiety represents a C₂₋₈ branched or linear alkyl group, C₃₋₈ branched or linear heteroalkyl group containing one heteroatom from the groups (N, O, S), a C₃₋₇ cycloalkyl group, C₃₋₇-cycloalkyl-C₁₋₃-alkyl group, C₃₋₇-heterocycloalkyl-C₁₋₃-alkyl group which groups may be substituted with a hydroxy, methoxy, methyl, trifluoromethylsulfonyl or trifluoromethyl group or a fluoro atom and which C₃₋₇-heterocycloalkyl-C₁₋₃-alkyl group contains one or two heteroatoms from the group (O, N, S), or said other moiety represents a benzyl group optionally substituted on its phenyl ring with 1-3 substituents Y, wherein Y has the abovementioned meaning,
- X represents one of the subgroups (i) or (ii), 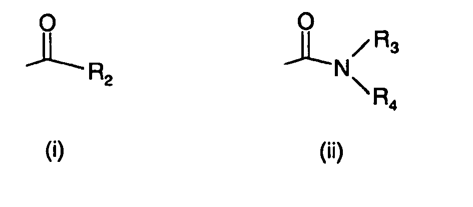 wherein:
- R₂ represents a C₃₋₈ branched or linear alkyl group, C₃₋₇ cycloalkyl group, C₃₋₇-cycloalkyl-C₁₋₂-alkyl group, C₃₋₇-heterocycloalkyl-C₁₋₂-alkyl group which groups may be substituted with a hydroxy, methyl or trifluoromethyl group or a fluoro atom and which C₃₋₇-heterocycloalkyl-C₁₋₂-alkyl group contains one or two heteroatoms from the group (O, N, S), or R₂ represents a phenyl, benzyl, phenethyl or phenylpropyl group which may be substituted on their phenyl ring with with 1-3 substituents Y, wherein Y has the abovementioned meaning, or R₂ represents a pyridyl, thienyl or naphtyl group, which napthyl group may be substituted with a halogen atom, a methyl group or a methoxy or trifluoromethyl group, with the proviso that when R₂ represents phenyl, R is not a 4-chlorophenyl group and excluding 2-benzoyl-4,5-diphenylthiazole, 2-benzoyl-4-p-methylphenyl-5-phenylthiazole, 2-benzoyl-4-p-chlorophenyl-5-phenylthiazole and 2-benzoyl-4-p-methoxyphenyl-5-phenylthiazole,
- R₃ represents a hydrogen atom or a branched or linear C₁₋₃ alkyl group,
- R₄ represents a branched or linear C₁₋₁₀ alkyl or C₃₋₈-cycloalkyl-C₁₋₂-alkyl group, branched or linear C₁₋₁₀ alkoxy, C₃₋₈ cycloalkyl, C₅₋₁₀ bicycloalkyl, C₅₋₁₀-bicycloalkyl-C₁₋₂-alkyl, C₆₋₁₀ tricycloalkyl, C₆₋₁₀ tricycloalkyl-methyl, branched or linear C₃₋₁₀ alkenyl, C₅₋₈ cycloalkenyl, which groups may contain one or more heteroatoms from the group (O, N, S) and which groups may be substituted with a hydroxy group, 1-3 methyl groups, an ethyl group or 1-3 fluoro atoms, or R₄ represents a phenyl, phenylamino, phenoxy, benzyl, phenethyl or phenylpropyl group, optionally substituted on their phenyl ring with 1-3 substituents Y, wherein
- Y has the abovementioned meaning, or R₄ represents a pyridyl or thienyl group, or R₄ represents a group NR₅R₆ wherein R₅ and R₆ - together with the nitrogen atom to which they are attached -form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, hydroxy or trifluoromethyl group or a fluoro atom, or
R₃ and R₄ - together with the nitrogen atom to which they are attached - form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, amino, hydroxy, methoxy, cyano or trifluoromethyl group or a fluoro or chloro atom,
and pharmacologically acceptable salts thereof.

3. A compound as claimed in claim 2, or a salt thereof, for use as a medicament.

4. Pharmaceutical compositions containing at least one compound as claimed in claim 2 as active ingredient.

5. Use of a compound as claimed in claim 2 for the preparation of a pharmaceutical composition for the treatment of psychosis, anxiety, depression, attention deficits, memory disorders, cognitive disorders, appetite disorders, obesity, addiction, appetence, drug dependence, neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, spinal cord injury, plaque sclerosis, viral encephalitis, demyelinisation related disorders, septic shock, glaucoma, cancer, diabetes, emesis, nausea, asthma, respiratory diseases, gastrointestinal disorders, gastric ulcers, diarrhoea and cardiovascular disorders.

6. A compound of general formula (IV) 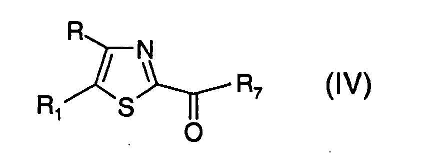 wherein one of R and R₁ represents a phenyl or 3-pyridinyl or 4-pyridinyl group, optionally substituted with 1-3 substituents Y, wherein Y has the abovementioned meaning and the other moiety represents a C₂₋₈ branched or linear alkyl group, C₃₋₈ branched or linear heteroalkyl group containing one heteroatom from the group (N, O, S) or an SO₂ group, C₃₋₇ cycloalkyl group, C₃₋₇-cycloalkyl-C₁₋₃-alkyl group, C₃₋₇-heterocycloalkyl-C₁₋₃-alkyl group which groups may be substituted with a hydroxy, methoxy, methyl, trifluoromethylsulfonyl or trifluoromethyl group or a fluoro atom and which C₃₋₇-heterocycloalkyl-C₁₋₃-alkyl group contains one or two heteroatoms from the group (O, N, S), or said other moiety represents a benzyl group optionally substituted on its phenyl ring with 1-3 substituents Y, wherein Y has the abovementioned meaning and R₇ represents a hydroxy, C₁₋₄ branched or linear alkoxy group or a benzyloxy group or a chloro atom or a N-methoxy-N-methylamino group, such compounds being useful in the synthesis of compounds of general formula (I).

## Patentansprüche

1. Verwendung einer Verbindung mit der Formel (I) 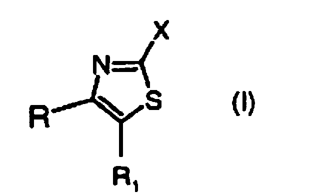 worin
- R und R₁ gleich oder verschieden sind und für eine Phenyl- oder Pyridinylgruppe stehen, optional substituiert mit 1-3 Substituenten Y, worin Y für einen Substituenten aus der Gruppe der Methyl-, Ethyl-, Propyl-, Methoxy-, Ethoxy-, Hydroxy-, Hydroxymethyl-, Hydroxyethyl-, Chlor-, Iod-, Brom-, Fluor-, Trifluormethyl-, Trifluormethoxy-, Methylsulfonyl-, Methylsulfanyl-, Trifluormethylsulfonyl-, Phenyl- oder Cyangruppen steht, mit der Maßgabe, dass X nicht für die Untergruppe (ii) steht,
oder einer der Reste R und R₁ für eine Phenyl- oder Pyridinylgruppe steht, optional substituiert mit 1-3 Substituenten Y, worin Y die zuvor genannte Bedeutung hat und der andere Rest für ein Wasserstoffatom oder eine verzweigte oder geradkettige C₁₋₈-Alkylgruppe, eine verzweigte oder geradkettige C₃₋₈-Heteroalkylgruppe enthaltend ein Heteroatom aus der Gruppe (N, O, S), eine C₃₋₇-Cycloalkylgruppe, eine C₃₋₇-Cycloalkyl-C₁₋₃-Alkylgruppe oder eine C₃₋₇-Heterocycloalkyl-C₁₋₃-Alkylgruppe steht, wobei diese Gruppen mit einer Hydroxy-, Methoxy-, Methyl-, Trifluormethylsulfonyl- oder Trifluormethylgruppe oder einem Fluoratom substituiert sein können und die C₃₋₇-Heterocycloalkyl-C₁₋₃-Alkylgruppe ein oder zwei Heteroatome aus der Gruppe (O, N, S) enthält oder der andere Rest für eine Benzylgruppe steht, deren Phenylring optional mit 1-3 Substituenten Y substituiert ist, worin Y die zuvor genannte Bedeutung hat,
- X für eine der Untergruppen (i) oder (ii) steht, 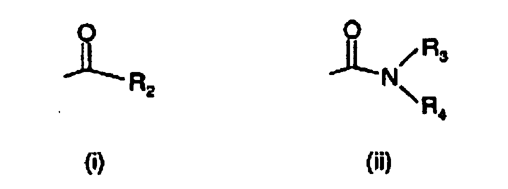 worin
R₂ für eine verzweigte oder geradkettige C₁₋₈-Alkylgruppe, eine C₃₋₇-Cycloalkylgruppe, eine C₃₋₇-Cycloalkyl-C₁₋₂-Alkylgruppe oder eine C₃₋₇-Heterocycloalkyl-C₁₋₂-Alkylgruppe steht, wobei diese Gruppen mit einer Hydroxy-, Methyl- oder Trifluormethylgruppe oder einem Fluoratom substituiert sein können und die C₃₋₇-Heterocycloalkyl-C₁₋₂-Alkylgruppe ein oder zwei Heteroatome aus der Gruppe (O, N, S) enthält, oder R₂ für eine Phenyl-, Benzyl-, Phenethyl- oder Phenylpropylgruppe steht, deren Phenylring mit 1-3 Substituenten Y substituiert sein kann, worin Y die zuvor genannte Bedeutung hat, oder R₂ für eine Pyridyl-, Thienyl- oder Naphthylgruppe steht, wobei die Naphthylgruppe mit einem Halogenatom, einer Methylgruppe oder einer Methoxy- oder Trifluormethylgruppe substituiert sein kann,
- R₃ für ein Wasserstoffatom oder eine verzweigte oder geradkettige C₁₋₃-Alkylgruppe steht,
- R₄ für Wasserstoff, eine verzweigte oder geradkettige C₁₋₁₀-Alkyl- oder C₃₋₈-Cycloalkyl-C₁₋₂-Alkylgruppe, eine verzweigte oder geradkettige C₁₋₁₀-Alkoxy-, C₃₋₈-Cycloalkyl-, C₅₋₁₀-Bicycloalkyl-, C₅₋₁₀-Bicycloalkyl-C₁₋₂-Alkyl-, C₆₋₁₀-Tricycloalkyl- oder C₆₋₁₀-Tricycloalkylmethylgruppe, eine verzweigte oder geradkettige C₃₋₁₀-Alkenyl- oder eine C₅₋₈-Cycloalkenylgruppe steht, wobei diese Gruppen ein oder mehrere Heteroatome aus der Gruppe (O, N, S) enthalten können und mit einer Hydroxygruppe, 1-3 Methylgruppen, einer Ethylgruppe oder 1-3 Fluoratomen substituiert sein können, oder R₄ für eine Phenyl-, Phenylamino-, Phenoxy-, Benzyl-, Phenethyl- oder Phenylpropylgruppe steht, deren Phenylring optional mit 1-3 Substituenten Y substituiert ist, wobei Y die zuvor genannte Bedeutung hat, oder R₄ für eine Pyridyl- oder Thienylgruppe steht, oder R₄ für eine Gruppe NR₅R₆ steht, worin
R₅ und R₆ - zusammen mit dem Stickstoffatom, an dem sie befestigt sind - eine gesättigte oder ungesättigte, monocyclische oder bicyclische, heterocyclische Gruppe mit 4 bis 10 Ringatomen bilden, wobei die heterocyclische Gruppe ein oder mehrere Heteroatome aus der Gruppe (O, N, S) enthält und mit einer verzweigten oder geradkettigen C₁₋₃-Alkyl-, Phenyl-, Hydroxy- oder Trifluormethylgruppe oder einem Fluoratom substituiert sein kann, oder
R₃ und R₄ - zusammen mit dem Stickstoffatom, an dem sie befestigt sind - eine gesättigte oder ungesättigte, monocyclische oder bicyclische, heterocyclische Gruppe mit 4 bis 10 Ringatomen bilden, wobei die heterocyclische Gruppe ein oder mehrere Heteroatome aus der Gruppe (O, N, S) enthält und mit einer verzweigten oder geradkettigen C₁₋₃-Alkyl-, Phenyl-, Amino-, Hydroxy-, Methoxy-, Cyan- oder Trifluormethylgruppe oder einem Fluor- oder Chloratom substituiert sein kann,
sowie deren pharmazeutisch verträglicher Salze zur Herstellung einer pharmazeutischen Zusammensetzung zur Behandlung von Psychose, Angstzuständen, Depression, Konzentrationsschwäche, Sucht, Appetenz, Drogenabhängigkeit, Dystonie, Muskelspastik, Tremor, Parkinson-Krankheit, Epilepsie, Chorea Huntigton, Tourette-Syndrom, Rückenmarksverletzungen, viraler Enzephalitis, Schmerzstörungen, septischem Schock, Glaukom, Krebs, Erbrechen, Übelkeit, sexuellen Störungen und Diarrhoe.

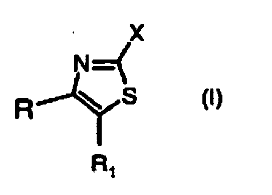

2. Verbindung mit der Formel (I)
worin
- R und R₁ gleich oder verschieden sind und für eine Phenyl-, 3-Pyridinyl- oder 4-Pyridinylgruppe stehen, optional substituiert mit 1-3 Substituenten Y, worin Y für einen Substituenten aus der Gruppe der Methyl-, Ethyl-, Propyl-, Methoxy-, Ethoxy-, Hydroxy-, Hydroxymethyl-, Hydroxyethyl-, Chlor-, Iod-, Brom-, Fluor-, Trifluormethyl-, Trifluormethoxy-, Methylsulfonyl-, Methylsulfanyl-, Trifluormethylsulfonyl-, Phenyl- oder Cyangruppen steht, mit der Maßgabe, dass X nicht für die Untergruppe (ii) steht,
oder einer der Reste R und R₁ für eine Phenyl-, 3-Pyridinyl- oder 4-Pyridinylgruppe steht, optional substituiert mit 1-3 Substituenten Y, worin Y die zuvor genannte Bedeutung hat und der andere Rest für eine verzweigte oder geradkettige C₂₋₈-Alkylgruppe, eine verzweigte oder geradkettige C₃₋₈-Heteroalkylgruppe enthaltend ein Heteroatom aus der Gruppe (N, O, S), eine C₃₋₇-Cycloalkylgruppe, eine C₃₋₇-Cycloalkyl-C₁₋₃-Alkylgruppe oder eine C₃₋₇-Heterocycloalkyl-C₁₋₃-Alkylgruppe steht, wobei diese Gruppen mit einer Hydroxy-, Methoxy-, Methyl-, Trifluormethylsulfonyl- oder Trifluormethylgruppe oder einem Fluoratom substituiert sein können und die C₃₋₇-Heterocycloalkyl-C₁₋₃-Alkylgruppe ein oder zwei Heteroatome aus der Gruppe (O, N, S) enthält oder der andere Rest für eine Benzylgruppe steht, deren Phenylring optional mit 1-3 Substituenten Y substituiert ist, worin Y die zuvor genannte Bedeutung hat, 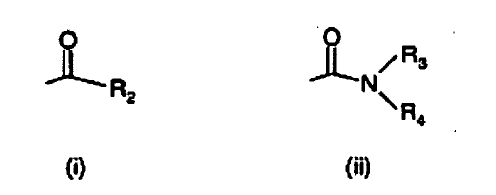
- X für eine der Untergruppen (i) oder (ii) steht,
worin
R₂ für eine verzweigte oder geradkettige C₃₋₈-Alkylgruppe, eine C₃₋₇-Cycloalkylgruppe, eine C₃₋₇-Cycloalkyl-C₁₋₂-Alkylgruppe oder eine C₃₋₇-Heterocycloalkyl-C₁₋₂-Alkylgruppe steht, wobei diese Gruppen mit einer Hydroxy-, Methyl- oder Trifluormethylgruppe oder einem Fluoratom substituiert sein können und die C₃₋₇-Heterocycloalkyl-C₁₋₂-Alkylgruppe ein oder zwei Heteroatome aus der Gruppe (O, N, S) enthält, oder R₂ für eine Phenyl-, Benzyl-, Phenethyl- oder Phenylpropylgruppe steht, deren Phenylring mit 1-3 Substituenten Y substituiert sein kann, worin Y die zuvor genannte Bedeutung hat, oder R₂ für eine Pyridyl-, Thienyl- oder Naphthylgruppe steht, wobei die Naphthylgruppe mit einem Halogenatom, einer Methylgruppe oder einer Methoxy- oder Trifluormethylgruppe substituiert sein kann, mit der Maßgabe, dass, wenn R₂ für eine Phenylgruppe steht, R keine 4-Chlorphenylgruppe ist und 2-Benzoyl-4,5-diphenylthiazol, 2-Benzoyl-4-p-methylphenyl-5-phenylthiazol, 2-Benzoyl-4-p-Chlorphenyl-5-phenylthiazol und 2-Benzoyl-4-p-methoxyphenyl-5-phenylthiazol ausgeschlossen sind,
- R₃ für ein Wasserstoffatom oder eine verzweigte oder geradkettige C₁₋₃-Alkylgruppe steht,
- R₄ für eine verzweigte oder geradkettige C₁₋₁₀-Alkyl- oder C₃₋₈-Cycloalkyl-C₁₋₂-Alkylgruppe, eine verzweigte oder geradkettige C₁₋₁₀-Alkoxy-, C₃₋₈-Cycloalkyl-, C₅₋₁₀-Bicycloalkyl-, C₅₋₁₀-Bicycloalkyl-C₁₋₂-Alkyl-, C₆₋₁₀-Tricycloalkyl- oder C₆₋₁₀-Tricycloalkylmethylgruppe, eine verzweigte oder geradkettige C₃-₁₀-Alkenyl- oder C₅₋₈-Cycloalkenylgruppe steht, wobei diese Gruppen ein oder mehrere Heteroatome aus der Gruppe (O, N, S) enthalten können und mit einer Hydroxygruppe, 1-3 Methylgruppen, einer Ethylgruppe oder 1-3 Fluoratomen substituiert sein können, oder R₄ für eine Phenyl-, Phenylamino-, Phenoxy-, Benzyl-, Phenethyl- oder Phenylpropylgruppe steht, deren Phenylring optional mit 1-3 Substituenten Y substituiert ist, wobei Y die zuvor genannte Bedeutung hat, oder R₄ für eine Pyridyl- oder Thienylgruppe steht, oder R₄ für eine Gruppe NR₅R₆ steht, worin
R₅ und R₆ - zusammen mit dem Stickstoffatom, an dem sie befestigt sind - eine gesättigte oder ungesättigte, monocyclische oder bicyclische, heterocyclische Gruppe mit 4 bis 10 Ringatomen bilden, wobei die heterocyclische Gruppe ein oder mehrere Heteroatome aus der Gruppe (O, N, S) enthält und mit einer verzweigten oder geradkettigen C₁₋₃-Alkyl-, Phenyl-, Hydroxy- oder Trifluormethylgruppe oder einem Fluoratom substituiert sein kann, oder
R₃ und R₄ - zusammen mit dem Stickstoffatom, an dem sie befestigt sind - eine gesättigte oder ungesättigte, monocyclische oder bicyclische, heterocyclische Gruppe mit 4 bis 10 Ringatomen bilden, wobei die heterocyclische Gruppe ein oder mehrere Heteroatome aus der Gruppe (O, N, S) enthält und mit einer verzweigten oder geradkettigen C₁₋₃-Alkyl-, Phenyl-, Amino-, Hydroxy-, Methoxy-, Cyan- oder Trifluormethylgruppe oder einem Fluor- oder Chloratom substituiert sein kann,
sowie pharmazeutisch verträgliche Salze davon.

3. Verbindung nach Anspruch 2 oder ein Salz davon zur Verwendung als Medikament.

4. Pharmazeutische Zusammensetzungen, die mindestens eine Verbindung nach Anspruch 2 als wirksamen Bestandteil enthalten.

5. Verwendung einer Verbindung nach Anspruch 2 zur Herstellung einer pharmazeutischen Zusammensetzung zur Behandlung von Psychose, Angstzuständen, Depression, Konzentrationsschwäche, Gedächtnisstörungen, kognitiven Störungen, Appetitstörungen, Adipositas, Sucht, Appetenz, Drogenabhängigkeit, neurodegenerativen Störungen, Demenz, Dystonie, Muskelspastik, Tremor, Epilepsie, multipler Sklerose, Hirntrauma, Schlaganfall, Parkinson-Krankheit, Alzheimer-Krankheit, Epilepsie, Chorea Huntigton, Tourette-Syndrom, zerebraler Ischämie, zerebraler Apoplexie, Schädel-Hirn-Trauma, Rückenmarksverletzungen, sklerotischer Plaque, viraler Enzephalitis, mit Demyelinisation assoziierten Störungen, septischem Schock, Glaukom, Krebs, Diabetes, Erbrechen, Übelkeit, Asthma, Atemwegserkrankungen, gastrointestinalen Störungen, Magengeschwüren, Diarrhoe und kardiovaskulären Störungen.

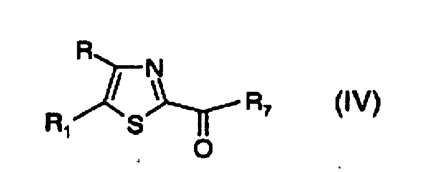

6. Verbindung mit der allgemeinen Formel (IV)
worin einer der Reste R oder R₁ für eine Phenyl-, 3-Pyridinyl- oder 4-Pyridinylgruppe steht, optional substituiert mit 1-3 Substituenten Y, worin Y die zuvor genannte Bedeutung hat und der andere Rest für eine verzweigte oder geradkettige C₂₋₈-Alkylgruppe, eine verzweigte oder geradkettige C₃₋₈-Heteroalkylgruppe enthaltend ein Heteroatom aus der Gruppe (N, O, S) oder eine SO₂-Gruppe, eine C₃₋₇-Cycloalkylgruppe, eine C₃₋₇-Cycloalkyl-C₁₋₃-Alkylgruppe oder eine C₃₋₇-Heterocycloalkyl-C₁₋₃-Alkylgruppe steht, wobei diese Gruppen mit einer Hydroxy-, Methoxy-, Methyl-, Trifluormethylsulfonyl- oder Trifluormethylgruppe oder einem Fluoratom substituiert sein können und die C₃₋₇-Heterocycloalkyl-C₁₋₃-Alkylgruppe ein oder zwei Heteroatome aus der Gruppe (O, N, S) enthält, oder der andere Rest für eine Benzylgruppe steht, deren Phenylring optional mit 1-3 Substituenten Y substituiert ist, worin Y die zuvor genannte Bedeutung hat und R₇ für eine Hydroxygruppe, eine verzweigte oder geradkettige C₁₋₄-Alkoxygruppe oder eine Benzyloxygruppe oder ein Chloratom oder eine N-Methoxy-N-methylaminogruppe steht, wobei solche Verbindungen nützlich bei der Synthese von Verbindungen der allgemeinen Formel (I) sind.

## Revendications

1. Utilisation d'un composé de formule (I) : 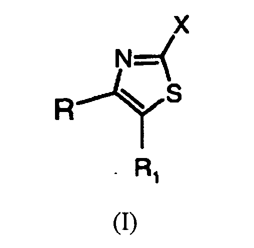 dans laquelle
R et R₁ sont identiques ou différents et représentent des radicaux phényle ou pyridinyle éventuellement substitués par 1 à 3 substituants Y, où Y représente un substituant choisi dans l'ensemble constitué par méthyle, éthyle, propyle, méthoxy, éthoxy, hydroxy, hydroxyméthyle, hydroxyéthyle, chloro, iodo, bromo, fluoro, trifluorométhyle, trifluorométhoxy, méthylsulfonyle, méthylsulfanyle, trifluorométhylsulfonyle, phényle et cyano, sous réserve que X ne représente pas le sous-groupe (ii),
ou bien l'un des fragments R et R₁ représente un groupe phényle ou pyridinyle éventuellement substitué par 1 à 3 substituants Y, où Y a la signification susmentionnée, et l'autre fragment représente un atome d'hydrogène ou un groupe alkyle en C₁ à C₈ linéaire ou ramifié, un groupe hétéroalkyle en C₃ à C₈ linéaire ou ramifié contenant un hétéroatome de l'ensemble (N, O, S), un groupe cycloalkyle en C₃ à C₇, un groupe (cycloalkyl en C₃ à C₇)-alkyle en C₁ à C₃, un groupe (hétérocycloalkyl en C₃ à C₇)-alkyle en C₁ à C₃, ces groupes pouvant être substitués par un groupe hydroxy, méthoxy, méthyle, trifluorométhylsulfonyle ou trifluorométhyle ou par un atome de fluor, et le groupe (hétérocycloalkyl en C₃ à C₇)-alkyle en C₁ à C₃ contenant un ou deux hétéroatomes de l'ensemble (O, N, S), ou bien ledit autre fragment représente un groupe benzyle éventuellement substitué sur son cycle phényle par 1 à 3 substituants Y, où Y a la signification susmentionnée,
X représente l'un des sous-groupes (i) et (ii), 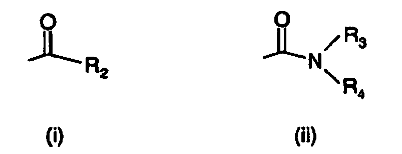 dans lesquels
R₂ représente un groupe alkyle en C₁ à C₈ linéaire ou ramifié, un groupe cycloalkyle en C₃ à C₇, un groupe (cycloalkyl en C₃ à C₇)-alkyle en C₁ à C₂, un groupe (hétérocycloalkyl en C₃ à C₇)-alkyle en C₁ à C₂, ces groupes pouvant être substitués par un groupe hydroxy, méthyle ou trifluorométhyle ou par un atome de fluor, et le groupe (hétérocycloalkyl en C₃ à C₇)-alkyle en C₁ à C₂ contenant un ou deux hétéroatomes de l'ensemble (O, N, S), ou bien R₂ représente un groupe phényle, benzyle, phénéthyle ou phénylpropyle qui peut être substitué sur le cycle phényle par 1 à 3 substituants Y, où Y a la signification susmentionnée, ou bien R₂ représente un groupe pyridyle, thiényle ou naphtyle, le groupe naphtyle pouvant être substitué par un atome d'halogène, un groupe méthyle ou un groupe méthoxy ou trifluorométhyle,
R₃ représente un atome d'hydrogène ou un groupe alkyle en C₁ à C₃ linéaire ou ramifié,
R₄ représente l'hydrogène, ou un groupe alkyle en C₁ à C₁₀ linéaire ou ramifié, (cycloalkyl en C₃ à C₈)-alkyle en C₁ à C₂, alcoxy en C₁ à C₁₀ linéaire ou ramifié, cycloalkyle en C₃ à C₈, bicycloalkyle en C₅ à C₁₀, (bicycloalkyl en C₅ à C₁₀)-alkyle en C₁ à C₂, tricycloalkyle en C₆ à C₁₀, (tricycloalkyl en C₆ à C₁₀)-méthyle, alcényle en C₃ à C₁₀ linéaire ou ramifié, cycloalcényle en C₅ à C₈, ces groupes pouvant contenir un ou plusieurs hétéroatomes de l'ensemble (O, N, S), et ces groupes pouvant être substitués par un groupe hydroxy, 1-3 groupes méthyle, un groupe éthyle ou 1-3 atomes de fluor, ou bien R₄ représente un groupe phényle, phénylamino, phénoxy, benzyle, phénéthyle ou phénylpropyle, éventuellement substitué sur le cycle phényle par 1 à 3 substituants Y, où Y a la signification susmentionnée, ou bien R₄ représente un groupe pyridyle ou thiényle, ou bien R₄ représente un groupe NR₅R₆ dans lequel
R₅ et R₆, conjointement avec l'atome d'azote auquel ils sont rattachés, forment un groupe hétérocyclique monocyclique ou bicyclique, saturé ou insaturé, ayant de 4 à 10 atomes sur le cycle, ce groupe hétérocyclique contenant un ou plusieurs hétéroatomes de l'ensemble (O, N, S), et ce groupe hétérocyclique pouvant être substitué par un groupe alkyle en C₁ à C₃ linéaire ou ramifié, phényle, hydroxy ou trifluorométhyle ou par un atome de fluor, ou bien R₃ et R₄, conjointement avec l'atome d'azote auquel ils sont rattachés, forment un groupe hétérocyclique monocyclique ou bicyclique, saturé ou insaturé, ayant de 4 à 10 atomes sur le cycle, ce groupe hétérocyclique contenant un ou plusieurs hétéroatomes de l'ensemble (O, N, S), et ce groupe hétérocyclique pouvant être substitué par un groupe alkyle en C₁ à C₃ linéaire ou ramifié, phényle, amino, hydroxy, méthoxy, cyano ou trifluoro-méthyle ou par un atome de fluor ou de chlore,
et de ses sels pharmacologiquement acceptables, pour la préparation d'une composition pharmaceutique destinée au traitement de la psychose, de l'anxiété, de la dépression, des déficits de l'attention, de l'attachement maladif, de l'appétence, de la pharmacodépendance, de la dystonie, de la spasticité musculaire, des tremblements, de la maladie de Parkinson, de l'épilepsie, de la maladie de Huntington, du syndrome de Tourette, d'une lésion de la moelle épinière, d'une encéphalite virale, de troubles liés à la douleur, du choc septique, du glaucome, d'un cancer, des vomissements, des nausées, des troubles sexuels et des diarrhées.

2. Composé de formule (I) 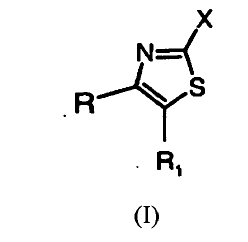 dans laquelle
R et R₁ sont identiques ou différents et représentent des radicaux phényle, 3-pyridinyle ou 4-pyridinyle éventuellement substitués par 1 à 3 substituants Y, où Y représente un substituant choisi dans l'ensemble constitué par méthyle, éthyle, propyle, méthoxy, éthoxy, hydroxy, hydroxyméthyle, hydroxyéthyle, chloro, iodo, bromo, fluoro, trifluorométhyle, trifluorométhoxy, méthyl-sulfonyle, méthylsulfanyle, trifluorométhylsulfonyle, phényle et cyano, sous réserve que X ne représente pas le sous-groupe (ii),
ou bien l'un des fragments R et R₁ représente un groupe phényle, 3-pyridinyle ou 4-pyridinyle éventuellement substitué par 1 à 3 substituants Y, où Y a la signification susmentionnée, et l'autre fragment représente un groupe alkyle en C₂ à C₈ linéaire ou ramifié, un groupe hétéroalkyle en C₃ à C₈ linéaire ou ramifié contenant un hétéroatome de l'ensemble (N, O, S), un groupe cycloalkyle en C₃ à C₇, un groupe (cycloalkyl en C₃ à C₇)-alkyle en C₁ à C₃, un groupe (hétérocycloalkyl en C₃ à C₇)-alkyle en C₁ à C₃, ces groupes pouvant être substitués par un groupe hydroxy, méthoxy, méthyle, trifluorométhylsulfonyle ou trifluorométhyle ou par un atome de fluor, et le groupe (hétérocycloalkyl en C₃ à C₇)-alkyle en C₁ à C₃ contenant un ou deux hétéroatomes de l'ensemble (O, N, S), ou bien ledit autre fragment représente un groupe benzyle éventuellement substitué sur son cycle phényle par 1 à 3 substituants Y, où Y a la signification susmentionnée,
X représente l'un des sous-groupes (i) et (ii), 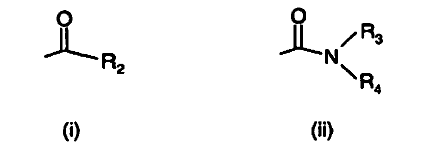 dans lesquels
R₂ représente un groupe alkyle en C₃ à C₈ linéaire ou ramifié, un groupe cycloalkyle en C₃ à C₇, un groupe (cycloalkyl en C₃ à C₇)-alkyle en C₁ à C₂, un groupe (hétérocycloalkyl en C₃ à C₇)-alkyle en C₁ à C₂, ces groupes pouvant être substitués par un groupe hydroxy, méthyle ou trifluorométhyle ou par un atome de fluor, et le groupe (hétérocycloalkyl en C₃ à C₇)-alkyle en C₁ à C₂ contenant un ou deux hétéroatomes de l'ensemble (O, N, S), ou bien R₂ représente un groupe phényle, benzyle, phénéthyle ou phénylpropyle qui peut être substitué sur le cycle phényle par 1 à 3 substituants Y, où Y a la signification susmentionnée, ou bien R₂ représente un groupe pyridyle, thiényle ou naphtyle, le groupe naphtyle pouvant être substitué par un atome d'halogène, un groupe méthyle ou un groupe méthoxy ou trifluorométhyle, sous réserve que, lorsque R₂ représente phényle, R ne soit pas un groupe 4-chlorophényle, et à l'exclusion du 2-benzoyl-4,5-diphénylthiazole, du 2-benzoyl-4-p-méthylphényl-5-phénylthiazole, du 2-benzoyl-4-p-chlorophényl-5-phényl-thiazole et du 2-benzoyl-4-p-méthoxyphényl-5-phényl-thiazole,
R₃ représente un atome d'hydrogène ou un groupe alkyle en C₁ à C₃ linéaire ou ramifié,
R₄ représente un groupe alkyle en C₁ à C₁₀ linéaire ou ramifié, (cycloalkyl en C₃ à C₈)-alkyle en C₁ à C₂, alcoxy en C₁ à C₁₀ linéaire ou ramifié, cycloalkyle en C₃ à C₈, bicycloalkyle en C₅ à C₁₀, (bicycloalkyl en C₅ à C₁₀)-alkyle en C₁ à C₂, tricycloalkyle en C₆ à C₁₀, (tricycloalkyl en C₆ à C₁₀)-méthyle, alcényle en C₃ à C₁₀ linéaire ou ramifié, cycloalcényle en C₅ à C₈, ces groupes pouvant contenir un ou plusieurs hétéroatomes de l'ensemble (O, N, S), et ces groupes pouvant être substitués par un groupe hydroxy, 1-3 groupes méthyle, un groupe éthyle ou 1-3 atomes de fluor, ou bien R₄ représente un groupe phényle, phénylamino, phénoxy, benzyle, phénéthyle ou phénylpropyle, éventuellement substitué sur le cycle phényle par 1 à 3 substituants Y, où Y a la signification susmentionnée, ou bien R₄ représente un groupe pyridyle ou thiényle, ou bien R₄ représente un groupe NR₅R₆ dans lequel R₅ et R₆, conjointement avec l'atome d'azote auquel ils sont rattachés, forment un groupe hétérocyclique monocyclique ou bicyclique, saturé ou insaturé, ayant de 4 à 10 atomes sur le cycle, ce groupe hétérocyclique contenant un ou plusieurs hétéroatomes de l'ensemble (O, N, S), et ce groupe hétérocyclique pouvant être substitué par un groupe alkyle en C₁ à C₃ linéaire ou ramifié, phényle, hydroxy ou trifluorométhyle ou par un atome de fluor, ou bien R₃ et R₄, conjointement avec l'atome d'azote auquel ils sont rattachés, forment un groupe hétérocyclique monocyclique ou bicyclique, saturé ou insaturé, ayant de 4 à 10 atomes sur le cycle, ce groupe hétérocyclique contenant un ou plusieurs hétéroatomes de l'ensemble (O, N, S), et ce groupe hétérocyclique pouvant être substitué par un groupe alkyle en C₁ à C₃ linéaire ou ramifié, phényle, amino, hydroxy, méthoxy, cyano ou trifluorométhyle ou par un atome de fluor ou de chlore,
et ses sels pharmacologiquement acceptables.

3. Composé selon la revendication 2, ou sel de celui-ci, pour une utilisation en tant que médicament.

4. Compositions pharmaceutiques contenant au moins un composé tel que revendiqué dans la revendication 2 à titre d'ingrédient actif.

5. Utilisation d'un composé tel que revendiqué dans la revendication 2 pour la préparation d'une composition pharmaceutique destinée au traitement de la psychose, de l'anxiété, de la dépression, des déficits de l'attention, des troubles de la mémoire, des troubles cognitifs, des troubles de l'appétit, de l'obésité, de l'attachement maladif, de l'appétence, de la pharmacodépendance, des troubles neurodégénératifs, de la démence, de la dystonie, de la spasticité musculaire, des tremblements, de l'épilepsie, de la sclérose en plaques, des lésions cérébrales traumatiques, des accidents cérébrovasculaires, de la maladie de Parkinson, de la maladie d'Alzheimer, de l'épilepsie, de la maladie de Huntington, du syndrome de Tourette, d'une ischémie cérébrale, d'une apoplexie cérébrale, d'un traumatisme crânio-cérébral, d'une lésion de la moelle épinière, de la sclérose en plaques, d'une encéphalite virale, de troubles liés à une démyélinisation, du choc septique, du glaucome, d'un cancer, du diabète, des vomissements, des nausées, de l'asthme, des maladies respiratoires, des troubles gastro-intestinaux, des ulcères gastriques, des diarrhées et des troubles cardiovasculaires.

6. Composé de formule générale (IV) 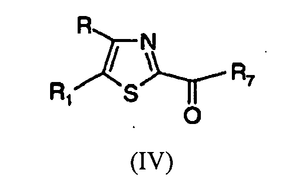 dans laquelle
l'un de R et R₁ représente un groupe phényle, 3-pyridinyle ou 4-pyridinyle éventuellement substitué par 1 à 3 substituants Y, où Y a la signification susmentionnée, et l'autre fragment représente un groupe alkyle en C₂ à C₈, linéaire ou ramifié, un groupe hétéroalkyle en C₃ à C₈ linéaire ou ramifié contenant un hétéroatome de l'ensemble (N, O, S) ou un groupe SO₂, un groupe cycloalkyle en C₃ à C₇, un groupe (cycloalkyl en C₃ à C₇)-alkyle en C₁ à C₃, un groupe (hétérocycloalkyl en C₃ à C₇)-alkyle en C₁ à C₃, ces groupes pouvant être substitués par un groupe hydroxy, méthoxy, méthyle, trifluorométhylsulfonyle ou trifluorométhyle ou par un atome de fluor, et le groupe (hétérocycloalkyl en C₃ à C₇)-alkyle en C₁ à C₃ contenant un ou deux hétéroatomes de l'ensemble (O, N, S), ou bien ledit autre fragment représente un groupe benzyle éventuellement substitué sur son cycle phényle par 1 à 3 substituants Y, où Y a la signification susmentionnée, et R₇ représente un groupe hydroxy, alcoxy en C₁ à C₄ linéaire ou ramifié ou benzyloxy ou un atome de chlore ou un groupe N-méthoxy-N-méthylamino, un tel composé étant utile dans la synthèse de composés de formule générale (I).
